# EUROPEAN PATENT SPECIFICATION

(11) **EP 4 506 375 B1**
(45) Date of publication and mention of the grant of the patent: **08.07.2026**
(21) Application number: 23928992.9
(22) Date of filing: 07.12.2023
(51) Int. Cl.: C08F 210/00, C08F 6/06, C08F 2/06

(54) **METHOD FOR PREPARING OLEFIN-BASED ELASTOMER**
VERFAHREN ZUR HERSTELLUNG EINES ELASTOMERS AUF OLEFINBASIS
PROCÉDÉ DE PRÉPARATION D'UN ÉLASTOMÈRE À BASE D'OLÉFINE

(30) Priority: 08.06.2023 KR 20230073348
(43) Date of publication of application: 12.02.2025
(73) Proprietor: LG Chem, Ltd., Seoul 07336 (KR)
(72) Inventor: JANG, Sung Keun, Daejeon 34122 (KR); JEONG, So Hyun, Daejeon 34122 (KR); CHOI, Seung Won, Daejeon 34122 (KR)
(74) Representative: Hoffmann Eitle
(86) International application number: PCT/KR2023/020061
(87) International publication number: WO 2024/253277

(56) References cited:
- KR-A- 20140 126 175
- KR-A- 20150 081 565
- KR-A- 20170 135 907
- KR-A- 20200 125 960
- KR-A- 20210 045 606

## Description

### [Technical Field]

### CROSS-REFERENCE TO RELATED APPLICATIONS

The present application claims the benefit of priority to Korean Patent Application No. 10-2023-0073348, filed on June 8, 2023.

### Technical Field

The present invention relates to a method for preparing an olefin-based elastomer, and more particularly, to a method for preparing an olefin-based elastomer by purifying and recycling a raw material recovered after preparing an olefin-based elastomer.

### [Background Art]

As an olefin-based compound has a lower density, it has elasticity like synthetic rubber, which is called an olefin-based elastomer. Since the olefin-based elastomer has excellent elasticity, processability, and impact strength, it is used not only as a reinforcement of physical properties such as impact and flexural strength of automobile interior and exterior materials but also in various fields such as materials for high-tech textiles, the sports industry, and extrusion coating.

In order to prepare the olefin-based elastomer as such, a solution polymerization method may be performed, and the solution polymerization method may be performed by dissolving a monomer and a comonomer in a solvent and copolymerizing them in the presence of a catalyst. After the olefin-based elastomer as a copolymer is finally obtained, a comonomer which is unreacted during the copolymerization may be included in a residual solvent.

Therefore, in order to reuse the residual solvent and the unreacted comonomer as a raw material for preparing the olefin-based elastomer, they are supplied to two or more distillation columns and purified to be separated into a solvent and a comonomer. As such, when purification is performed in two or more distillation columns, energy usage is excessively increased due to the use of a plurality of distillation columns.

KR 2015 0081565 A discloses an apparatus for polymerization of ethylene and alpha-olefin, comprising a polymerization reactor in which ethylene and alpha-olefin are supplied as reaction raw materials along with a solvent, and the reaction raw materials are copolymerized in a solution state, to thereby produce a polymerization product comprising an ethylene and alpha-olefin copolymer dissolved in the solvent; a unit for separating unreacted ethylene, the alpha-olefin, low molecular weight oligomer and a polymer, including a flash tower for distilling and separating the unreacted ethylene and alpha-olefin contained in the polymerization product, and a stripper unit for distilling and separating the solvent and the low molecular weight oligomer having a lower molecular weight than the molecular weight of the ethylene and alpha-olefin copolymer contained in the polymerization product; and a solvent recovery unit for separating the low molecular weight oligomer from the separated low molecular weight oligomer and solvent and recovering the solvent.

KR 2021 0045606 A discloses an apparatus for preparing an oligomer, including a reactor to which a monomer stream and a solvent stream are supplied to carry out oligomerization; and a first line and a second line provided at the lower lateral side of the reactor, while being spaced apart from each other, wherein the first line includes a first level-controlling valve, and the second line includes a second level-controlling valve.

KR 2020 0125960 A discloses a method for separating hydrogen from a gaseous feed stream in a polymerization process.

KR 2014 0126175 A discloses a method for continuous solution polymerization of olefins and a reaction device therefor.

KR 2017 0135907 A discloses a continuous solution polymerization process.

### [Disclosure]

### [Technical Problem]

In order to solve the problems mentioned in the Background Art, an object of the present invention is to provide a method for preparing an olefin-based elastomer which may simplify the process, reduce energy usage, and use purified raw materials, by recovering raw materials which have been used in the preparation of the olefin-based elastomer, specifically a solvent and an unreacted olefin comonomer and purifying them in the distillation column of the present invention.

### [Technical Solution]

In one general aspect, a method for preparing an olefin-based elastomer includes: supplying a cycle stream including an unreacted olefin comonomer and a residual solvent, a solvent stream including a solvent, and a comonomer stream including an olefin comonomer to a distillation column; obtaining an upper discharge stream from the distillation column including a low-boiling point component, a lower discharge stream from the distillation column including a high-boiling point component, and a side discharge stream from the distillation column including an olefin comonomer and the solvent, by distillation performed in the distillation column; supplying the side discharge stream from the distillation column and a monomer stream including an olefin monomer to a polymerization reactor and performing copolymerization to obtain a discharge stream from the polymerization reactor including an olefin-based elastomer solution; and supplying the discharge stream from the polymerization reactor including the olefin-based elastomer solution to a flash drum to obtain an olefin-based elastomer,
wherein the distillation column above is a single-area distillation column which does not include a structure dividing an area in a horizontal direction inside the distillation column.

### [Advantageous Effects]

According to the method for preparing an olefin-based elastomer, one distillation column is used in purifying a residual solvent recovered after a copolymerization reaction of an olefin monomer and an olefin comonomer, an unreacted olefin comonomer, a fresh solvent, and an olefin comonomer, thereby further reducing energy usage as compared with a conventional method using a plurality of distillation columns, and the method may be preferred in economic terms such as process equipment costs. Specifically, a weight ratio of a solvent to an olefin comonomer in a side discharge stream from the distillation column and a lateral position in the distillation column from which the side discharge stream is discharged of the present invention are controlled, thereby performing the purification which is conventionally performed by a plurality of distillation columns for purifying a recovered residual solvent, an unreacted olefin comonomer, a fresh solvent, and an olefin comonomer, by the distillation column design of the present invention. In addition, by the control, a content of a high-boiling point component in the side discharge stream from the distillation column may be reduced to prevent fouling occurrence, and the side discharge stream from the distillation column may be directly supplied to a polymerization reactor to activate a copolymerization reaction performed in the polymerization reactor.

### [Description of Drawings]

FIGS. 1 and 2 are process flow diagrams showing a method for preparing an olefin-based elastomer according to an exemplary embodiment of the present invention.
FIGS. 3 and 4 are process flow diagrams showing a method for preparing an olefin-based elastomer according to the comparative examples of the present invention.

### [Best Mode]

The term "stream" in the present invention may refer to a fluid flow in a process, or may refer to a fluid itself flowing in a pipe. Specifically, the stream may refer to both a fluid itself flowing in a pipe connecting each device and a fluid flow. In addition, the fluid may refer to a gas or liquid, and a case in which a solid substance is included in the fluid is not excluded.

Meanwhile, in the devices such as a distillation column and a flash drum in the present invention, unless otherwise particularly stated, a "lower portion" of the device refers to a point at a height of 80 % to 100% from top to bottom of the device, specifically the lowest part (bottom). Likewise, unless otherwise particularly stated, an "upper portion" of the device refers to, a point at a height of 0% to 20 % from top to bottom of the device, specifically the highest part (top).

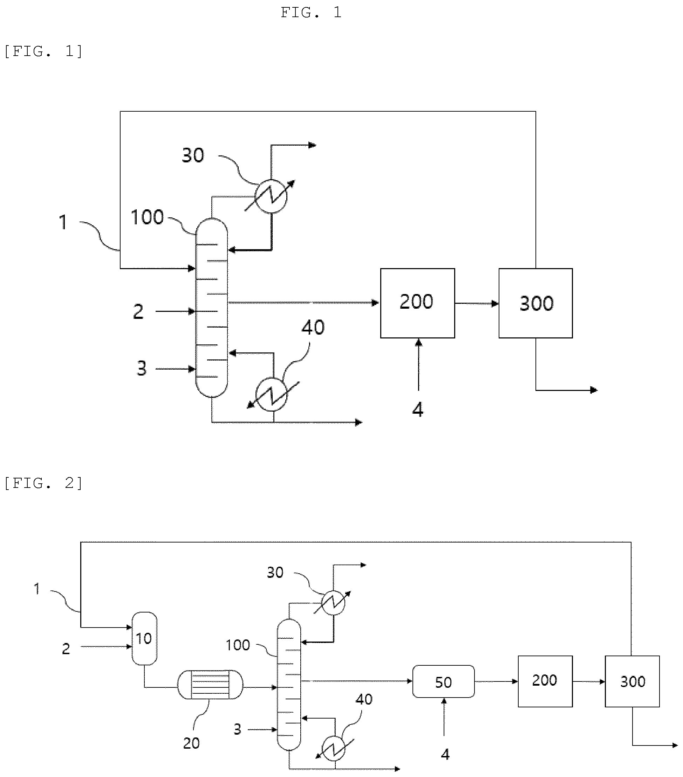

Hereinafter, the present invention will be described in more detail for better understanding of the present invention, with reference to FIG. 1.

A method for preparing an olefin-based elastomer according to an exemplary embodiment may include: supplying a cycle stream 1 including an unreacted olefin comonomer and a residual solvent, a solvent stream 2 including a solvent, and a comonomer stream 3 including an olefin comonomer to a distillation column 100; obtaining an upper discharge stream from the distillation column including a low-boiling point component, a lower discharge stream from the distillation column including a high-boiling point component, and a side discharge stream from the distillation column including an olefin comonomer and the solvent, by distillation performed in the distillation column 100; supplying the side discharge stream from the distillation column and a monomer stream 4 including an olefin monomer to a polymerization reactor 200 and performing copolymerization to obtain a discharge stream from the polymerization reactor including an olefin-based elastomer solution; and supplying the discharge stream from the polymerization reactor including the olefin-based elastomer solution to a flash drum 300 to obtain an olefin-based elastomer, wherein the distillation column above is a single-area distillation column which does not include a structure dividing an area in a horizontal direction inside the distillation column.

First, the copolymerization reaction according to an exemplary embodiment of the present invention may be performed by copolymerizing a reactant including an olefin monomer and an olefin comonomer in the presence of a solvent, and an olefin-based elastomer which is a copolymer may be obtained by copolymerization of the reactant.

Herein, the olefin monomer may be any one selected from α-olefin monomers, cyclic olefin monomers, diene olefin monomers, triene olefin monomers, and styrene monomers, and in the present invention, may be an α-olefin monomer. More specifically, the α-olefin monomer may include one or more of ethylene and propylene.

Meanwhile, the olefin comonomer may be an α-olefin comonomer. Specifically, the α-olefin comonomer may include any one or a mixture of two or more selected from the group consisting of 1-butene, 1-pentene, 4-methyl-1-pentene, 1-hexene, 1-heptene, 1-octene, 1-decene, 1-undecene, 1-dodecene, 1-tetradecene, 1-hexadecene, 1-itocene, norbornene, norbornadiene, ethylidene norbornene, phenylnorbornene, vinylnorbornene, dicyclopentadiene, 1,4-butadiene, 1,5-pentadiene, 1,6-hexadiene, styrene, α-methylstyrene, divinylbenzene, and 3-chloromethylstyrene. The olefin comonomer in the present invention may be 1-butene or 1-octene.

More specifically, when the olefin comonomer is 1-butene, the reactant may include, for example, ethylene and 1-butene, and when the olefin comonomer is 1-octene, the reactant may include, for example, ethylene and 1-octene.

First, the method for preparing an olefin-based elastomer may be performed by including: supplying a cycle stream 1 including an unreacted olefin comonomer and a residual solvent, a solvent stream 2 including a solvent, and a comonomer stream 3 including an olefin comonomer to a distillation column 100.

The cycle stream 1 including an unreacted olefin comonomer and a residual solvent may be derived from a stream separated from an olefin-based elastomer in a flash drum 300 described later. The unreacted olefin comonomer and the residual solvent may be an olefin comonomer which is not copolymerized during copolymerization of an olefin monomer and an olefin comonomer, and a solvent used in the copolymerization, which are separated from the olefin-based elastomer in the flash drum 300. In order to reuse raw materials used in the copolymerization, purification in which the cycle stream 1 including the unreacted olefin comonomer and the residual solvent is supplied to the distillation column 100 to separate impurities included in the cycle stream 1 may be performed.

Meanwhile, the solvent stream 2 including the solvent is a stream including a fresh solvent, and may be a stream for freshly supplementing a solvent required for copolymerization of the olefin monomer and the olefin comonomer.

Specifically, the solvent may be a hydrocarbon-based solvent having 5 to 12 carbon atoms, and specifically, may be one or more of hexane, cyclohexane, methylcyclohexane, heptane, nonane, decane, toluene, and benzene. More specifically, the solvent may be hexane. When the olefin monomer and the olefin comonomer are copolymerized using the solvent, the olefin comonomer and the olefin monomer may be more easily dissolved by the solvent, and thus, copolymerization of the olefin monomer and the olefin comonomer dissolved thereby may be more easily performed.

Meanwhile, the comonomer stream 3 including the olefin comonomer is a stream including a fresh olefin comonomer, and may be a stream for freshly supplementing the olefin comonomer which is a raw material used in preparing the olefin-based elastomer into the polymerization reactor 200.

Meanwhile, in the comonomer stream 3, undesired impurities may be included in addition to the fresh olefin comonomer. When the comonomer stream 3 is supplied to the polymerization reactor 200 without removing impurities included in the comonomer stream and a copolymerization reaction is performed, a side reaction may occur during copolymerization due to the impurities, and an amount of the olefin-based elastomer obtained by the copolymerization may be decreased. Therefore, the impurities included in the comonomer stream 3 needs to be removed in advance and supplied to the polymerization reactor 200. Herein, when a separate column for removing only the impurities included in the comonomer stream 3 is provided, it may be economically not preferred due to increased process equipment costs and excessive energy use. Therefore, it may be preferred to supply the comonomer stream 3 to the distillation column 100 in which the cycle stream 1 is purified and purify them together.

Meanwhile, conventionally, there was no choice but to perform purification using a plurality of distillation columns in order to separate impurities included in the cycle stream 1, the solvent stream 2, and the comonomer stream 3. However, in the present invention, a weight ratio between the solvent and the olefin comonomer in the side discharge stream from the distillation column and the height of the side portion of the distillation column from which the side discharge stream is discharged are controlled, thereby performing purification of the cycle stream 1, the solvent stream 2, and the comonomer stream 3 only by the distillation column 100 according to the present invention.

Meanwhile, the cycle stream 1, the solvent stream 2, and the comonomer stream 3 may be directly supplied to the distillation column 100, but the supply of the cycle stream 1, the solvent stream 2, and the comonomer stream 3 to the distillation column 100 may be variously modified.

For example, according to another exemplary embodiment of the present invention (see FIG. 2), the supply of the cycle stream 1, the solvent stream 2, and the comonomer stream 3 may be performed by mixing the cycle stream 1 and the solvent stream 2 and supplying the mixture to the distillation column 100, and directly supplying the comonomer stream 3 to the distillation column 100. Mixing of the cycle stream 1 and the solvent stream 2 may be performed in a recovery tank 10.

In addition, mixing the cycle stream 1 and the solvent stream 2 and supplying the mixture to the distillation column 100 may be supplying a mixed stream of the cycle stream 1 and the solvent stream 2 to the distillation column through the heat exchanger 20. Specifically, the mixed stream may be mixing the cycle stream 1 and the solvent stream 2 in the recovery tank 10. The mixed stream may pass through the heat exchanger 20, and in the heat exchanger 20, the mixed stream may be preheated using a low pressure steam (LS). As such, the mixed stream preheated in the heat exchanger 20 is supplied to the distillation column 100, thereby further reducing the usage of a medium pressure steam (MS) used in the reboiler 40 of the distillation column 100, which may be preferred in terms of energy use. Furthermore, the medium pressure steam is a higher grade steam than the low pressure steam used in the heat exchanger 20, and the usage of the medium pressure steam which is a higher grade steam is reduced, thereby improving process economic feasibility.

The method for preparing an olefin-based elastomer according to an exemplary embodiment of the present invention may be performed by including: obtaining an upper discharge stream from the distillation column including a low-boiling point component, a lower discharge stream from the distillation column including a high-boiling point component, and a side discharge stream from the distillation column including an olefin comonomer and a solvent, by distillation performed in the distillation column 100.

Specifically, separation into an upper fraction including the low-boiling point component, a lower fraction including a high-boiling point component, and a side fraction including an olefin comonomer and a solvent may be performed by distillation performed in the distillation column 100.

The low-boiling point component may include nitrogen, hydrogen, methane, ethane, ethylene, and propane, and the high-boiling point component may include isooctene, C9 or higher carbon compounds, and oligomers (molecular weight: 1000 to 10000 g/mol). The upper fraction including the low-boiling point component may be discharged through the upper discharge stream from the distillation column, and the lower fraction including the high-boiling point component may be discharged through the lower discharge stream from the distillation column. As such, impurities are discharged through the upper discharge stream from the distillation column and the lower discharge stream from the distillation column, thereby removing impurities included in the cycle stream 1, the solvent stream 2, and the comonomer stream 3.

As described above, the impurities are separated through the upper discharge stream from the distillation column and the lower discharge stream from the distillation column and the side discharge stream from the distillation column including the side fraction is obtained, whereby the solvent and the olefin comonomer included in the side discharge stream from the distillation column may be appropriate for use as a raw material for preparing an olefin-based elastomer.

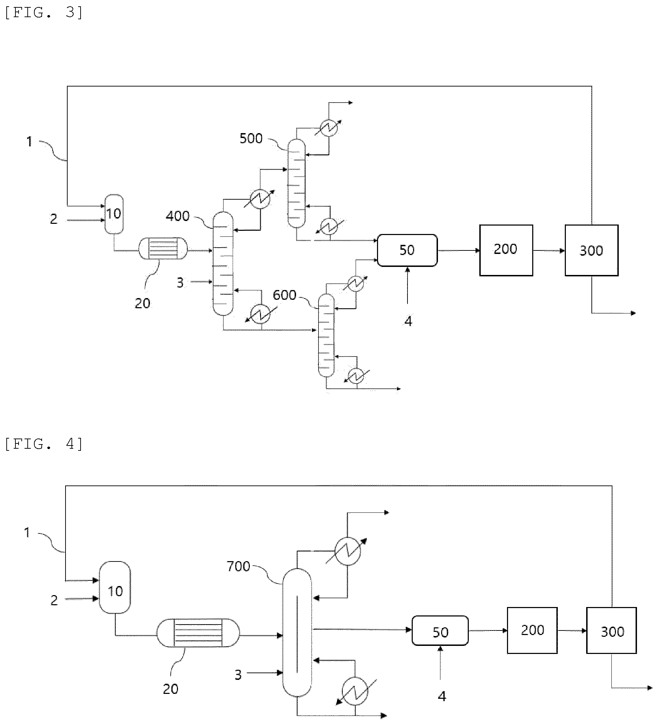

Meanwhile, referring to FIG. 3, conventionally, the monomer and the comonomer are copolymerized in the presence of a solvent to obtain a copolymer, and a residual solvent including an unreacted comonomer is recovered, which is distilled with a fresh solvent and a fresh olefin comonomer in the distillation column to separate the solvent and the comonomer, respectively. Further, in order to reuse the solvent and the comonomer which are separated, respectively, as such, as a raw material of the copolymer, two or more distillation columns for removing the low-boiling point component included in the separated solvent and the high-boiling point component included in the separated comonomer are further needed. However, when a solvent and a comonomer from which impurities have been removed are obtained by a total of 3 or more distillation columns as such, process equipment costs and energy usage are excessively increased due to operation of a plurality of distillation columns.

Unlike the conventional process of removing impurities using at least 3 or more distillation columns, the present invention functionally uses one distillation column 100 including discharge of a side stream, thereby purifying a fresh solvent, a fresh olefin comonomer, a recovered residual solvent, and an unreacted olefin comonomer to obtain a solvent and an olefin comonomer from which a low-boiling point component and a high-boiling point component have been removed. Specifically, a weight ratio between the solvent and the olefin comonomer in the side discharge stream from the distillation column, and a height of the side portion of the distillation column from which the side discharge stream is discharged are controlled, thereby activating separation and removal of the low-boiling point component and the high-boiling point component only with the distillation column according to an exemplary embodiment of the present invention. Thus, it may be preferred in terms of energy and economic feasibility as compared with the conventional process. In addition, the solvent and the comonomer are not separated, respectively, as before, and the side discharge stream from the distillation column including the solvent and the olefin comonomer may be directly supplied to the polymerization reactor 200.

According to an exemplary embodiment, the weight ratio of the solvent to the olefin comonomer included in the side discharge stream from the distillation column may be 1.28 to 1.58, specifically 1.28 to 1.48 or 1.28 to 1.37. When the weight ratio of the solvent to the olefin comonomer included in the side discharge stream from the distillation column is within the range, it may be appropriate to supply the side discharge stream from the distillation column to the polymerization reactor 200 to prepare an olefin-based elastomer. That is, when the weight ratio of the solvent to the olefin comonomer included in the side discharge stream from the distillation column is less than 1.28, the flow rate of the lower discharge stream from the distillation column is very low or there is no flow rate, so that the distillation column 100 may not be operated, and even in the case in which the distillation column 100 may be operated, the content of the high-boiling point component in the side discharge stream from the distillation column is increased to cause fouling from the high-boiling point component. When the weight ratio of the solvent to the olefin comonomer included in the side discharge stream from the distillation column is more than 1.58, the recovery rate of the olefin comonomer from the side discharge stream from the distillation column is decreased, so that it may be difficult to activate copolymerization performed in the polymerization reactor 200. Therefore, a yield of the olefin-based elastomer to be obtained in the present invention may be decreased.

The side discharge stream from the distillation column may be discharged from the side portion at a height of 60 to 77%, 62 to 73%, or 66 to 70% from top to bottom of the distillation column. When the height of the side portion of the distillation column from which the side discharge stream is discharged is within the range, the weight ratio of the solvent to the olefin comonomer included in the side discharge stream from the distillation column may be easily controlled. Specifically, the weight ratio of the solvent to the olefin comonomer included in the side discharge stream from the distillation column may be controlled by flow rates of the solvent stream 2 and the comonomer stream **3,** but it may be difficult to flexibly respond the flow rates of the solvent stream 2 and the comonomer stream 3 depending on the process needs, for example, product yield and product grade within a limited flow rate range. Therefore, the weight ratio of the solvent to the olefin comonomer included in the side discharge stream from the distillation column may be controlled to 1.28 to 1.58 by controlling the height of the side portion of the distillation column from which the side discharge stream is discharged.

Specifically, when the height of the side portion of the distillation column from which the side discharge stream is discharged is less than 60%, the weight of the low-boiling point component in the side discharge stream from the distillation column may be increased, and when the height of the side portion of the distillation column from which the side discharge stream is discharged is more than 77%, the weight of the high-boiling point component in the side discharge stream from the distillation column may be increased. That is, when the height of the side portion of the distillation column from which the side discharge stream is discharged is out of the range (60 to 77%), the content of impurities in the side discharge stream from the distillation column is excessively increased, and thus, when the side discharge stream from the distillation column is supplied to the polymerization reactor 200, copolymerization of the olefin monomer and the olefin comonomer performed in the polymerization reactor 200 may not be activated.

The distillation column 100 of the present invention is a conventional distillation column, and the conventional distillation column is a single area distillation column which does not include a structure dividing an area in a horizontal direction inside the distillation column. Specifically, since the distillation column 100 of the present invention does not include the structure dividing an area in the horizontal direction, for example, a left area and a right area which have different composition operating temperatures and operation pressures in the distillation column may not be divided. For example, the distillation column 100 may be a distillation column which does not have a separation wall.

According to the present invention, a temperature in the lower portion of the distillation column may be 120 to 180°C, specifically 130 to 170°C or 149 to 156°C. When the lower portion of the distillation column is controlled to the temperature within the range, the amount of the solvent and/or olefin comonomer lost through the upper discharge stream from the distillation column and the lower discharge stream from the distillation column may be minimized. Therefore, since the amount of the lost solvent and/or olefin comonomer is small, the solvent and the olefin comonomer may be included as much as possible in the side discharge stream from the distillation column.

Meanwhile, the pressure of the upper pressure of the distillation column may be 0.7 to 1.0 kg/cm²·g, specifically 0.75 to 0.95 kg/cm²·g, or 0.85 to 0.9 kg/cm²·g. When distillation is performed under the pressure conditions of the upper portion of the distillation column as such, separation of the upper fraction including the low-boiling point component, the lower fraction including the high-boiling point component, and the side fraction including the solvent and the olefin comonomer may be easily performed in the distillation column 100.

Meanwhile, an upper reflux ratio of the flow rate of the upper reflux stream from the distillation column to the flow rate of the upper discharge stream from the distillation column may be 60 to 75, specifically 62 to 70 or 64 to 68. The upper discharge stream from the distillation column passes through a condenser 30, and a part is refluxed to the distillation column 100 and the rest is discharged, in which the part of the stream refluxed to the distillation column 100 may be the upper reflux stream from the distillation column.

In addition, a lower reflux ratio of the flow rate of the lower reflux stream from the distillation column to the flow rate of the lower discharge stream from the distillation column may be 20 to 220, specifically 22 to 215, or 24 to 213. A part of the lower discharge stream from the distillation column passes through a reboiler 40 and is refluxed to the distillation column 100 and the rest is discharged, in which the part of the stream refluxed to the distillation column 100 may be the lower reflux stream from the distillation column.

The method for preparing an olefin-based elastomer according to an exemplary embodiment of the present invention may include: supplying the side discharge stream from the distillation column and a monomer stream 4 including the olefin monomer to the polymerization reactor 200 and performing copolymerization to obtain a discharge stream from the polymerization reactor including an olefin-based elastomer solution. The monomer stream 4 may be a stream for supplying an olefin monomer which is a raw material used for preparing an olefin-based elastomer in the polymerization reactor 200, and may include a fresh olefin monomer.

Meanwhile, the side discharge stream from the distillation column and the monomer stream 4 may be cooled through a freezer directly before supplying to the polymerization reactor 200. Herein, when the content of the high-boiling point component in the side discharge stream from the distillation column is high, an oligomer included in the high-boiling point component is precipitated at a low temperature to cause fouling from the oligomer, which may reduce efficiency of the copolymerization reaction.

According to the present invention, supply of the side discharge stream from the distillation column and the monomer stream 4 may be performed by direct supply to the polymerization reactor 200, as described above, but may be performed by mixing the side discharge stream from the distillation column and the monomer stream 4 and then supplying a mixture of the side discharge stream from the distillation column and the monomer stream to the polymerization reactor 200. Herein, mixing of the side discharge stream from the distillation column and the monomer stream 4 may be performed by supplying the side discharge stream from the distillation column and the monomer stream 4 to a feed tank 50.

According to an exemplary embodiment of the present invention, a weight flow ratio of the olefin comonomer to the olefin monomer supplied to the polymerization reactor 200 may be 0.8 to 1.5, 0.85 to 1.4, or 0.9 to 1.3. The weight flow ratio of the olefin comonomer to the olefin monomer supplied to the polymerization reactor 200 is controlled to the range, thereby adjusting the physical properties of the olefin-based elastomer which is the final product to be obtained in the present invention to a desired level.

When the weight flow ratio of the olefin comonomer to the olefin monomer supplied to the polymerization reactor 200 is less than 0.8, the olefin comonomer more than necessary is supplied to the polymerization reactor 200, which may not be economically preferred in terms of raw material utilization. In addition, the content of the olefin comonomer in the finally obtained olefin-based elastomer may be increased to decrease density which is one of quality elements of the final product. When the weight flow ratio of the olefin comonomer to the olefin monomer supplied to the polymerization reactor 200 is more than 1.5, the amount of the olefin comonomer supplied to the polymerization reactor 200 is not sufficient to decrease the efficiency of the copolymerization reaction performed in the polymerization reactor 200 and excessively increase the density of the final product, so that an olefin-based elastomer having desired physical properties may not be obtained.

Meanwhile, in the polymerization reactor 200, a reactant including the olefin monomer and the olefin comonomer may be copolymerized in the presence of a solvent to obtain an olefin-based elastomer solution including an olefin-based elastomer which is a copolymer. Herein, the polymerization reactor 200 may further include a catalyst and a cocatalyst, and the catalyst may include a metallocene catalyst and the cocatalyst may include one or more of triethylaluminoxane (TEAL), methylaluminoxane (MAO), and borate.

The method of copolymerizing the reactant may be performed by liquid, slurry, bulk phase, or gaseous polymerization, and in the present invention, may be performed by liquid polymerization, that is, solution polymerization. The solution polymerization is a method of polymerization in a solution state by dissolving a monomer in a solvent, and after the polymerization is completed, a polymer solution in the form of a polymer dissolved or dispersed in the solvent may be obtained.

Meanwhile, a temperature at which the olefin monomer and the olefin comonomer are copolymerized in the polymerization reactor 200 may be 140 to 180°C, and a pressure of copolymerization may be 80 to 100 kg/cm²·g. When the polymerization reactor 200 is operated under the operation conditions, copolymerization of the olefin monomer and the olefin comonomer is easily performed to increase a yield of the olefin-based elastomer.

The method for preparing an olefin-based elastomer according to an exemplary embodiment of the present invention may be performed by including: supplying a discharge stream from the polymerization reactor including the olefin-based elastomer solution to a flash drum 300 to obtain an olefin-based elastomer. Specifically, the olefin-based elastomer which is a copolymer of the olefin monomer and the olefin comonomer may be separately obtained from the olefin-based elastomer solution in the flash drum 300. Herein, the olefin-based elastomer may be an ultralow density polyolefin having a density of 0.875 g/cm³ or less.

Herein, the flash drum 300 may be operated under the operation conditions of a temperature of 160 to 240°C and a pressure of 10 to 17 kg/cm²·g. When the olefin-based elastomer is separated under the operation conditions of the flash drum 300, the olefin-based elastomer is more easily separated in the flash drum 300, and the cycle stream 1 derived from the stream separated from the olefin-based elastomer may be obtained.

As described above, the cycle stream 1 derived from the stream separated from the olefin-based elastomer in the flash drum 300 may include a residual solvent and an unreacted olefin comonomer. The residual solvent and the unreacted olefin comonomer included in the cycle stream 1 are purified in the distillation column 100 and reused as a raw material for preparing the olefin-based elastomer, thereby minimizing the amounts of the solvent and the olefin comonomer which should be freshly supplied in the process. Therefore, since process costs for supplying a fresh raw material may be reduced, the method may be economically preferred.

Hereinafter, the present invention will be described in more detail by the examples. However, the following examples are provided for illustrating the present invention only.

### Examples

### Example 1

According to the process flow diagram illustrated in FIG. 1, a process of preparing an olefin-based elastomer was simulated, using an Aspen Plus simulator available from Aspen Technology, Inc.

Specifically, a cycle stream 1 including an unreacted olefin comonomer (unreacted 1-octene) and a residual solvent (residual hexane), a solvent stream 2 including a fresh solvent (hexane), and a comonomer stream 3 including a fresh olefin comonomer (1-octene) were supplied to a distillation column 100.

An upper discharge stream from the distillation column including a low-boiling point component, a lower discharge stream from the distillation column including a high-boiling point component, and a side discharge stream from the distillation column including an olefin comonomer and a solvent were obtained by distillation performed in the distillation column 100. At this time, the temperature in the lower portion of the distillation column was 149°C, and the pressure in the upper portion of the distillation column was 0.82 kg/cm²·g. A reflux ratio of the flow rate of the upper reflux stream from the distillation column to the flow rate of the upper discharge stream from the distillation column was 66.7, and the reflux ratio of the flow rate of the lower reflux stream from the distillation column to the flow rate of the lower discharge stream from the distillation column was 32.6.

Meanwhile, the side discharge stream from the distillation column was discharged from the side portion at a height of 67% from top to bottom of the distillation column 100. A weight ratio of the solvent to the olefin comonomer included in the side discharge stream from the distillation column was 1.34. In addition, the content of the high-boiling point component (content of impurities) included in the side discharge stream from the distillation column was 0.2 ppm. At this time, the composition of the side discharge stream from the distillation column was measured by Fourier transform infrared spectroscopy (FT-IR).

The side discharge stream from the distillation column and monomer stream 4 including a fresh olefin monomer (ethylene) was supplied to the polymerization reactor 200 and copolymerized in the presence of a metallocene catalyst and a cocatalyst, thereby obtaining a discharge stream from the polymerization reactor including the olefin-based elastomer solution. Herein, the weight flow ratio of the olefin comonomer to the olefin monomer supplied to the polymerization reactor 200 was 0.96. At this time, the polymerization reactor 200 was operated at a temperature of 170°C and a pressure of 95 kg/cm²·g.

The discharge stream from the polymerization reactor including the olefin-based elastomer solution was supplied to a flash 300 to finally obtain an olefin-based elastomer. At this time the flash drum 300 was operated at a temperature of 200°C and a pressure of 10 kg/cm²·g. The cycle stream 1 including the unreacted olefin comonomer and the residual solvent derived from the stream separated from the olefin-based elastomer in the flash drum 300 was circulated to a recovery tank 10.

As a result, an energy reduction rate in a reboiler 40 in the distillation column 100 was 51%, and the energy reduction rate is a value of a difference in an energy usage in the reboiler between Comparative Example 1 and Example 1, expressed as a percentage, based on Comparative Example 1 (3.73Gcal/h) of which the total energy usage in the reboiler in the distillation column was the highest.

Meanwhile, a recovery rate in the distillation column 100 was 99.4 wt% in the case of hexane and 94.5 wt% in the case of 1-octene. The recovery rate of hexane was a weight ratio of hexane included in the side discharge stream from the distillation column to hexane included in the cycle stream 1 and the solvent stream 2, expressed as a percentage. The recovery rate of 1-octene was a weight ratio of 1-octene included in the side discharge stream from the distillation column to 1-octene included in the cycle stream 1 and the comonomer stream 3, expressed as a percentage.

### Example 2

According to the process flow diagram illustrated in FIG. 2, a process of preparing an olefin-based elastomer was simulated, using an Aspen Plus simulator available from Aspen Technology, Inc.

Specifically, the cycle stream 1 including an unreacted olefin comonomer (unreacted 1-octene) and a residual solvent (residual hexane) and the solvent stream 2 including a fresh solvent (hexane) were supplied to the recovery tank 10 and mixed to obtain a mixed stream of the cycle stream 1 and the solvent stream 2, and the mixed stream was passed through a heat exchanger 20 and supplied to the distillation column 100. Meanwhile, the comonomer stream 3 including the fresh olefin comonomer (1-octene) was directly supplied to the distillation column 100.

An upper discharge stream from the distillation column including a low-boiling point component, a lower discharge stream from the distillation column including a high-boiling point component, and a side discharge stream from the distillation column including an olefin comonomer and a solvent were obtained by distillation performed in the distillation column 100. At this time, the temperature in the lower portion of the distillation column was 149°C, and the pressure in the upper portion of the distillation column was 0.82 kg/cm²·g. A reflux ratio of the flow rate of the upper reflux stream from the distillation column to the flow rate of the upper discharge stream from the distillation column was 66.7, and the reflux ratio of the flow rate of the lower discharge stream from the distillation column to the flow rate of the lower discharge stream from the distillation column was 32.6.

Meanwhile, the side discharge stream from the distillation column was discharged from the side portion at a height of 67% from top to bottom of the distillation column 100. A weight ratio of the solvent to the olefin comonomer included in the side discharge stream from the distillation column was 1.34. In addition, the content of the high-boiling point component (content of impurities) included in the side discharge stream from the distillation column was 3.0 ppm. At this time, the composition of the side discharge stream from the distillation column was measured by Fourier transform infrared spectroscopy (FT-IR).

The monomer stream 4 including the side discharge stream from the distillation column and the fresh olefin monomer (ethylene) was supplied to the feed tank 50, mixed, and supplied to the polymerization reactor 200. In the polymerization reactor 200, the olefin monomer and the olefin comonomer were copolymerized in the presence of a metallocene catalyst and a cocatalyst to obtain a discharge stream from the polymerization reactor including the olefin-based elastomer solution. At this time, the polymerization reactor 200 was operated at a temperature of 175°C and a pressure of 90 kg/cm²·g. Herein, the weight flow ratio of the olefin comonomer to the olefin monomer supplied to the polymerization reactor 200 was 0.96.

The discharge stream from the polymerization reactor including the olefin-based elastomer solution was supplied to a flash 300 to finally obtain an olefin-based elastomer. At this time, the flash drum 300 was operated at a temperature of 200°C and a pressure of 10 kg/cm²·g. The cycle stream 1 including the unreacted olefin comonomer and the residual solvent derived from the stream separated from the olefin-based elastomer in the flash drum 300 was circulated to a recovery tank 10.

As a result, the energy reduction rate in the reboiler 40 of the distillation column 100 was 64%. Meanwhile, the recovery rate in the distillation column 100 was 99.5% in the case of hexane and 94.6% in the case of 1-octene.

### Example 3

In Example 3, an olefin-based elastomer was prepared in the same process flow as in Example 2, except that the weight ratio of the solvent to the olefin comonomer included in the side discharge stream from the distillation column was 1.40. The content of impurities (high-boiling point component) included in the side discharge stream from the distillation column was 1.5 ppm.

As a result, the energy reduction rate in the reboiler 40 of the distillation column 100 was 64%. Meanwhile a recovery rate in the distillation column 100 was 99.4 wt% in the case of hexane and 90.7 wt% in the case of 1-octene.

### Example 4

In Example 4, an olefin-based elastomer was prepared in the same process flow as in Example 2, except that the weight ratio of the solvent to the olefin comonomer included in the side discharge stream from the distillation column was 1.27. The content of impurities (high-boiling point component) included in the side discharge stream from the distillation column was 362 ppm.

As a result, the energy reduction rate in the reboiler 40 of the distillation column 100 was 64%. Meanwhile, the recovery rate in the distillation column 100 was 99.5 wt% in the case of hexane and 99.0 wt% in the case of 1-octene.

### Example 5

In Example 5, an olefin-based elastomer was prepared in the same process flow as in Example 2, except that the weight ratio of the solvent to the olefin comonomer included in the side discharge stream from the distillation column was 1.28. The content of impurities (high-boiling point component) included in the side discharge stream from the distillation column was 25 ppm.

As a result, the energy reduction rate in the reboiler 40 of the distillation column 100 was 64%. Meanwhile, the recovery rate in the distillation column 100 was 99.5 wt% in the case of hexane and 99.4 wt% in the case of 1-octene.

### Comparative Examples

### Comparative Example 1

According to the process flow diagram illustrated in FIG. 3, a process of preparing an olefin-based elastomer was simulated, using an Aspen Plus simulator available from Aspen Technology, Inc.

A cycle stream 1 including an unreacted olefin comonomer (unreacted 1-octene) and a residual solvent (residual hexane) and the solvent stream 2 including a fresh solvent (hexane) were supplied to the recovery tank 10 and mixed to obtain a mixed stream of the cycle stream 1 and the solvent stream 2, and the mixed stream was passed through a heat exchanger 20 and supplied to a first distillation column 400. Meanwhile, the comonomer stream 3 including the fresh olefin comonomer (1-octene) was directly supplied to the first distillation column 400.

An upper discharge stream from the first distillation column including a low-boiling point component and the solvent and a lower discharge stream from the first distillation column including a high-boiling point component and an olefin comonomer were obtained by distillation performed in the first distillation column 400. At this time, the temperature in the lower portion of the first distillation column was 136°C, and the pressure in the upper portion of the first distillation column was 0.82 kg/cm²·g.

The upper discharge stream from the first distillation column was supplied to a second distillation column 500 and separation into an upper fraction including the low-boiling point component and a lower fraction including the solvent was performed. The lower fraction including the solvent was supplied to a feed tank 50 through the lower discharge stream from the second distillation column. At this time, the temperature in the lower portion of the second distillation column was 95°C, and the pressure in the upper portion of the second distillation column was 0.82 kg/cm²·g.

Meanwhile, the lower discharge stream from the first distillation column was supplied to a third distillation column 600 and separation into an upper fraction including the olefin comonomer and a lower fraction including the high-boiling point component was performed. The upper fraction including the olefin comonomer was supplied to the feed tank 50 through the upper discharge stream from the third distillation column. At this time, the temperature in the lower portion of the third distillation column was 145°C, and the pressure in the upper portion of the third distillation column was 0.4 kg/cm³·g. A weight ratio of the solvent included in the lower discharge stream from the second distillation column to the olefin comonomer included in the upper discharge stream from the third distillation column was 1.2. In addition, the content of the total high-boiling point component (content of impurities) included in the lower discharge stream from the second distillation column and the upper discharge stream from the third distillation column was 25 ppm.

In addition to the upper discharge stream from the third distillation column and the lower discharge stream from the second distillation column, a monomer stream 4 including a fresh olefin monomer (ethylene) was supplied to the feed tank 50, mixed, and supplied to the polymerization reactor 200. Herein, the weight flow ratio of the olefin comonomer to the olefin monomer supplied to the polymerization reactor 200 was 0.96. In the polymerization reactor 200, the olefin monomer and the olefin comonomer were copolymerized in the presence of a metallocene catalyst and a cocatalyst to obtain an olefin-based elastomer solution. At this time, the polymerization reactor 200 was operated at a temperature of 175°C and a pressure of 90 kg/cm²·g.

The olefin-based elastomer solution was supplied to the flash drum 300 to finally obtain an olefin-based elastomer. At this time the flash drum 300 was operated at a temperature of 200°C and a pressure of 10 kg/cm²·g. The cycle stream 1 including the unreacted olefin comonomer and the residual solvent derived from the stream separated from the olefin-based elastomer in the flash drum 300 was circulated to a recovery tank 10.

As a result, a total energy usage in the reboiler of the distillation columns (first distillation column, second distillation column, and third distillation column) was 3.73 Gcal/h.

Meanwhile, a recovery rate in the distillation columns (first distillation column, second distillation column, and third distillation column) was 87.8 wt% in the case of hexane and 93.5 wt% in the case of 1-octene. The recovery rate of hexane was a weight ratio of hexane included in the lower discharge stream from the second distillation column to hexane included in the cycle stream 1 and the solvent stream 2, expressed as a percentage. The recovery rate of 1-octene was a weight ratio of 1-octene included in the upper discharge stream from the third distillation column to 1-octene included in the cycle stream 1 and the comonomer stream 3, expressed as a percentage.

### Comparative Example 2

According to the process flow diagram illustrated in FIG. 4, a process of preparing an olefin-based elastomer was simulated, using an Aspen Plus simulator available from Aspen Technology, Inc.

A cycle stream 1 including an unreacted olefin comonomer (unreacted 1-octene) and a residual solvent (residual hexane) and the solvent stream 2 including a fresh solvent (hexane) were supplied to the recovery tank 10 and mixed to obtain a mixed stream of the cycle stream 1 and the solvent stream 2, and the mixed stream was passed through a heat exchanger 20 and supplied to a separation wall-type distillation column 700. Meanwhile, the comonomer stream 3 including the fresh olefin comonomer (1-octene) was directly supplied to the separation wall-type distillation column 700.

An upper discharge stream from the separation wall-type distillation column including a low-boiling point component, a side discharge stream from the separation wall-type distillation column including a solvent (hexane) and an olefin comonomer (1-octene), and a lower discharge stream from the separation wall-type distillation column including a high-boiling point component were obtained by distillation performed in the separation wall-type distillation column 700. The side discharge stream from the separation wall-type distillation column was discharged from the side portion at a height of 65 % from top to bottom of the separation wall-type distillation column 700. A weight ratio of the solvent to the olefin comonomer included in the side discharge stream from the separation wall-type distillation column was 1.33. At this time, the temperature in the lower portion of the separation wall-type distillation column was 150°C, and the pressure in the upper portion of the separation wall-type distillation column was 0.82 kg/cm²·g.

Meanwhile, the side discharge stream from the separation wall-type distillation column was supplied to a feed tank 50, and besides, a monomer stream 4 including a fresh olefin monomer (ethylene) was supplied to the feed tank 50, mixed, and supplied to the polymerization reactor 200.

In the polymerization reactor 200, the olefin monomer and the olefin comonomer were copolymerized in the presence of a metallocene catalyst and a cocatalyst to obtain an olefin-based elastomer solution including an olefin-based elastomer which is a copolymer. At this time, the polymerization reactor 200 was operated at a temperature of 175°C and a pressure of 90 kg/cm²·g.

The olefin-based elastomer solution was supplied to the flash drum 300 to finally obtain an olefin-based elastomer. At this time the flash drum 300 was operated at a temperature of 200°C and a pressure of 10 kg/cm²·g. The cycle stream 1 including the unreacted olefin comonomer and the residual solvent derived from the stream separated from the olefin-based elastomer in the flash drum 300 was circulated to a recovery tank 10.

As a result, the energy reduction rate in the reboiler of the separation wall-type distillation column 700 was 64%.

Meanwhile, a recovery rate in the separation wall-type distillation column 700 was 99.4 wt% in the case of hexane and 95.6 wt% in the case of 1-octene. The recovery rate of hexane was a weight ratio of hexane included in the side discharge stream from the separation wall-type distillation column to hexane included in the cycle stream 1 and the solvent stream 2, expressed as a percentage. The recovery rate of 1-octene was a weight ratio of 1-octene included in the side discharge stream from the separation wall-type distillation column to 1-octene included in the cycle stream 1 and the comonomer stream 3, expressed as a percentage.

The following Table 1 shows each of the operation conditions (weight ratio), the energy reduction rate, the recovery rate, and the content of impurities of the examples and the comparative examples.

**[Table 1]**

| | Weight ratio¹⁾ | Energy reduction rate (reboiler) | Recovery rate (wt%) | Content of impurities (high-boiling point component) (ppm) |
|---|---|---|---|---|
| Example 1 | 1.34 | 51% | Hexane: 99.4 | 0.2 |
| | | | 1-octene: 94.5 | |
| Example 2 | 1.34 | 64% | Hexane: 99.5 | 3.0 |
| | | | 1-octene: 94.6 | |
| Example 3 | 1.4 | 64% | Hexane: 99.4 | 1.5 |
| | | | 1-octene: 90.7 | |
| Example 4 | 1.27 | 64% | Hexane: 99.5 | 362 |
| | | | 1-octene: 99.0 | |
| Example 5 | 1.28 | 64% | Hexane: 99.5 | 25 |
| | | | 1-octene: 99.4 | |
| Compara tive Example 1 | 1.2 | 3.73 Gcal/h | Hexane: 87.8 | 47 |
| | | | 1-octene: 93.5 | |
| Compara tive Example 2 | 1.33 | 64% | Hexane: 99.4 | 568 |
| | | | 1-octene: 95.6 | |
| ¹⁾ The weight ratio was a weight ratio of the solvent to the olefin comonomer included in the side discharge stream from the distillation column in the examples, a weight ratio of the solvent included in the lower discharge stream from the second distillation column to the olefin comonomer included in the upper discharge stream from the third distillation column in Comparative Example 1, and a weight ratio of the solvent to the olefin comonomer included in the side discharge stream from the separation wall-type distillation column in Comparative Example 2, respectively. | | | | |

Referring to Table 1, in the examples using one distillation column for purifying the cycle stream, the solvent stream, and the comonomer stream, it was confirmed that the energy reduction rate was significantly increased as compared with Comparative Example 1. Meanwhile, in Examples 2 to 4, it was confirmed that the mixed stream of the cycle stream and the solvent stream was passed through the heat exchanger, thereby decreasing the amount of energy used in the reboiler in the distillation column as compared with Example 1 to increase the energy reduction rate. In addition, in Example 2, it was confirmed that the mixed stream was supplied to the distillation column in a preheated state via the heat exchanger, thereby increasing the amount of the high-boiling point component present in the side fraction of the distillation column as compared with Example 1 to increase the content of impurities included in the side discharge stream from the distillation column as compared with Example 1. However, when the content of impurities (high-boiling point component) included in the side discharge stream from the distillation column was less than 50 ppm, a problem such as fouling did not occur since the impurities were in a small amount.

However, in Comparative Example 1, it was confirmed that the total energy usage in the reboiler in each distillation column was the highest by using 3 distillation columns, and the content of impurities was increased as compared with Examples 1 to 3 and 5.

Meanwhile, Comparative Example 2 used a separation wall-type distillation column, not a general distillation column as in the examples, and it was confirmed that the content of impurities was the highest.

## Claims

1. Method for preparing an olefin-based elastomer, the method comprising: supplying a cycle stream including an unreacted olefin comonomer and a residual solvent, a solvent stream including a solvent, and a comonomer stream including an olefin comonomer to a distillation column; obtaining an upper discharge stream from the distillation column including a low-boiling point component, a lower discharge stream from the distillation column including a high-boiling point component, and a side discharge stream from the distillation column including an olefin comonomer and the solvent, by distillation performed in the distillation column; supplying the side discharge stream from the distillation column and a monomer stream including an olefin monomer to a polymerization reactor and performing copolymerization to obtain a discharge stream from the polymerization reactor including an olefin-based elastomer solution; and supplying the discharge stream from the polymerization reactor including the olefin-based elastomer solution to a flash drum to obtain an olefin-based elastomer,
wherein the distillation column above is a single-area distillation column which does not include a structure dividing an area in a horizontal direction inside the distillation column.

2. The method for preparing an olefin-based elastomer of claim 1, wherein the cycle stream including the unreacted olefin comonomer and the residual solvent is derived from a stream separated from the olefin-based elastomer in the flash drum.

3. The method for preparing an olefin-based elastomer of claim 1, wherein the olefin monomer includes one or more of ethylene and propylene.

4. The method for preparing an olefin-based elastomer of claim 1, wherein the olefin comonomer includes 1-butene or 1-octene.

5. The method for preparing an olefin-based elastomer of claim 1, wherein the solvent includes one or more of cyclohexane, methylcyclohexane, heptane, nonane, decane, toluene, benzene, and hexane.

6. The method for preparing an olefin-based elastomer of claim 1, wherein the supplying of a cycle stream, a solvent stream, and a comonomer stream is performed by mixing the cycle stream and the solvent stream and supplying the mixture to the distillation column, and directly supplying the comonomer stream to the distillation column.

7. The method for preparing an olefin-based elastomer of claim 6, wherein the mixing of the cycle stream and the solvent stream and the supplying of the mixture to the distillation column are supplying a mixed stream of the cycle stream and the solvent stream to the distillation column via a heat exchanger.

8. The method for preparing an olefin-based elastomer of claim 1, wherein a weight ratio of the solvent to the olefin comonomer included in the side discharge stream from the distillation column is 1.28 to 1.58.

9. The method for preparing an olefin-based elastomer of claim 1, wherein a side discharge stream from the distillation column is discharged from a side portion at a height of 60% to 77% from top to bottom of the distillation column.

10. The method for preparing an olefin-based elastomer of claim 1, wherein a A
weight flow ratio of the olefin comonomer to the olefin monomer supplied to the polymerization reactor is 0.8 to 1.5.

11. The method for preparing an olefin-based elastomer of claim 1, wherein a temperature in a lower portion of the distillation column is 120 to 180°C.

12. The method for preparing an olefin-based elastomer of claim 1, wherein a pressure in an upper portion of the distillation column is 0.7 to 1.0 kg/cm²·g.

13. The method for preparing an olefin-based elastomer of claim 1, wherein an upper reflux ratio of a flow rate of an upper reflux stream from the distillation column to a flow rate of an upper discharge stream from the distillation column is 60 to 75.

14. The method for preparing an olefin-based elastomer of claim 1, wherein a lower reflux ratio of a flow rate of a lower reflux stream from the distillation column to a flow rate of a lower discharge stream from the distillation column is 20 to 220.

15. The method for preparing an olefin-based elastomer of claim 1, wherein the supplying of the side discharge stream from the distillation column and the monomer stream is performed by mixing the side discharge stream from the distillation column and the monomer stream and supplying a mixture of the side discharge stream from the distillation column and the monomer stream to the polymerization reactor.

## Patentansprüche

1. Verfahren zur Herstellung eines Elastomers auf Olefinbasis, wobei das Verfahren umfasst:
Zuführen eines Zyklusstroms, der ein nicht umgesetztes Olefin-Comonomer und ein Restlösungsmittel enthält, eines Lösungsmittelstroms, der ein Lösungsmittel enthält, und eines Comonomerstroms, der ein Olefin-Comonomer enthält, zu einer Destillationskolonne;
Gewinnen eines oberen Austrittsstroms aus der Destillationskolonne, der eine Komponente mit niedrigem Siedepunkt enthält, eines unteren Austrittsstroms aus der Destillationskolonne, der eine Komponente mit hohem Siedepunkt enthält, und eines Seitenaustrittsstroms aus der Destillationskolonne, der ein Olefin-Comonomer und das Lösungsmittel enthält, mittels in der Destillationskolonne durchgeführte Destillation;
Zuführen des Seitenaustrittsstroms aus der Destillationskolonne und eines Monomerstroms, der ein Olefinmonomer enthält, zu einem Polymerisationsreaktor und Durchführen einer Copolymerisation, um einen Austrittsstrom aus dem Polymerisationsreaktor zu erhalten, der eine Elastomerlösung auf Olefinbasis enthält; und
Zuführen des Austrittsstroms aus dem Polymerisationsreaktor, der die Elastomerlösung auf Olefinbasis enthält, zu einer Entspannungstrommel (flash drum), um ein Elastomer auf Olefinbasis zu erhalten,
wobei die obige Destillationskolonne eine Einbereichs-Destillationskolonne ist, die keine Struktur aufweist, die einen Bereich in horizontaler Richtung innerhalb der Destillationskolonne unterteilt.

2. Verfahren zur Herstellung eines Elastomers auf Olefinbasis gemäß Anspruch 1, wobei der Zyklusstrom, der das nicht umgesetzte Olefin-Comonomer und das Restlösungsmittel enthält, aus einem Strom stammt, der in der Entspannungstrommel vom Elastomer auf Olefinbasis abgetrennt wurde.

3. Verfahren zur Herstellung eines Elastomers auf Olefinbasis gemäß Anspruch 1, wobei das Olefinmonomer eines oder mehrere enthält aus Ethylen und Propylen.

4. Verfahren zur Herstellung eines Elastomers auf Olefinbasis gemäß Anspruch 1, wobei das Olefin-Comonomer 1-Buten oder 1-Octen enthält.

5. Verfahren zur Herstellung eines Elastomers auf Olefinbasis gemäß Anspruch 1, wobei das Lösungsmittel eines oder mehrere enthält aus Cyclohexan, Methylcyclohexan, Heptan, Nonan, Decan, Toluol, Benzol und Hexan.

6. Verfahren zur Herstellung eines Elastomers auf Olefinbasis gemäß Anspruch 1, wobei das Zuführen eines Zyklusstroms, eines Lösungsmittelstroms und eines Comonomerstroms ausgeführt wird, indem der Zyklusstrom und der Lösungsmittelstrom gemischt werden und die Mischung der Destillationskolonne zugeführt wird und der Comonomerstrom direkt der Destillationskolonne zugeführt wird.

7. Verfahren zur Herstellung eines Elastomers auf Olefinbasis gemäß Anspruch 6, wobei das Mischen des Zyklusstroms und des Lösungsmittelstroms sowie das Zuführen der Mischung zu der Destillationskolonne darin bestehen, einen gemischten Strom aus dem Zyklusstrom und dem Lösungsmittelstrom der Destillationskolonne über einen Wärmetauscher zuzuführen.

8. Verfahren zur Herstellung eines Elastomers auf Olefinbasis gemäß Anspruch 1, wobei das Gewichtsverhältnis von Lösungsmittel zu Olefin-Comonomer, das in dem Seitenaustrittsstrom aus der Destillationskolonne enthalten ist, 1,28 bis 1,58 beträgt.

9. Verfahren zur Herstellung eines Elastomers auf Olefinbasis gemäß Anspruch 1, wobei ein Seitenaustrittsstrom aus der Destillationskolonne aus einem Seitenabschnitt in einer Höhe von 60 % bis 77 % von oben nach unten der Destillationskolonne abgegeben wird.

10. Verfahren zur Herstellung eines Elastomers auf Olefinbasis gemäß Anspruch 1, wobei ein Gewichtsflussverhältnis des Olefin-Comonomers zu dem Olefinmonomer, das dem Polymerisationsreaktor zugeführt wird, 0,8 bis 1,5 beträgt.

11. Verfahren zur Herstellung eines Elastomers auf Olefinbasis gemäß Anspruch 1, wobei die Temperatur in einem unteren Abschnitt der Destillationskolonne 120 bis 180 °C beträgt.

12. Verfahren zur Herstellung eines Elastomers auf Olefinbasis gemäß Anspruch 1, wobei der Druck in einem oberen Abschnitt der Destillationskolonne 0,7 bis 1,0 kg/cm²·g beträgt.

13. Verfahren zur Herstellung eines Elastomers auf Olefinbasis gemäß Anspruch 1, wobei das obere Rückflussverhältnis von einer Durchflussrate eines oberen Rückflussstroms aus der Destillationskolonne zur Durchflussrate eines oberen Austrittsstroms aus der Destillationskolonne 60 bis 75 beträgt.

14. Verfahren zur Herstellung eines Elastomers auf Olefinbasis gemäß Anspruch 1, wobei das untere Rückflussverhältnis von einer Durchflussrate eines unteren Rückflussstroms aus der Destillationskolonne zur Durchflussrate eines unteren Austrittsstroms aus der Destillationskolonne 20 bis 220 beträgt.

15. Verfahren zur Herstellung eines Elastomers auf Olefinbasis gemäß Anspruch 1, wobei das Zuführen des Seitenaustrittsstroms aus der Destillationskolonne und des Monomerstroms durchgeführt werden, indem der Seitenaustrittsstrom aus der Destillationskolonne und der Monomerstrom gemischt werden und eine Mischung aus dem Seitenaustrittsstrom aus der Destillationskolonne und dem Monomerstrom dem Polymerisationsreaktor zugeführt wird.

## Revendications

1. Procédé de préparation d'un élastomère à base d'oléfine, le procédé comprenant les étapes consistant à : fournir un flux de recyclage incluant un comonomère oléfinique non réagi et un solvant résiduel, un flux de solvant incluant un solvant, et un flux de comonomère incluant un comonomère oléfinique à une colonne de distillation ;
obtenir un flux de sortie de tête provenant de la colonne de distillation incluant un composant à point d'ébullition bas, un flux de sortie de fond provenant de la colonne de distillation incluant un composant à point d'ébullition élevé, et un flux de sortie latéral provenant de la colonne de distillation incluant un comonomère oléfinique et le solvant, par la distillation mise en œuvre dans la colonne de distillation ;
fournir le flux de sortie latéral provenant de la colonne de distillation et un flux de monomère incluant un monomère oléfinique à un réacteur de polymérisation et mettre en œuvre une copolymérisation pour obtenir un flux de sortie provenant du réacteur de polymérisation incluant une solution d'élastomère à base d'oléfine ;
et fournir le flux de sortie provenant du réacteur de polymérisation incluant la solution d'élastomère à base d'oléfine à un ballon de détente pour obtenir un élastomère à base d'oléfine,
dans lequel la colonne de distillation ci-dessus est une colonne de distillation à zone unique qui n'inclut pas une structure divisant une zone dans une direction horizontale à l'intérieur de la colonne de distillation.

2. Procédé de préparation d'un élastomère à base d'oléfine selon la revendication 1, dans lequel le flux de recyclage incluant le comonomère oléfinique non réagi et le solvant résiduel est dérivé d'un flux séparé de l'élastomère à base d'oléfine dans le ballon de détente.

3. Procédé de préparation d'un élastomère à base d'oléfine selon la revendication 1, dans lequel le monomère oléfinique inclut un ou plusieurs éléments parmi l'éthylène et le propylène.

4. Procédé de préparation d'un élastomère à base d'oléfine selon la revendication 1, dans lequel le comonomère oléfinique inclut du 1-butène ou du 1-octène.

5. Procédé de préparation d'un élastomère à base d'oléfine selon la revendication 1, dans lequel le solvant inclut un ou plusieurs éléments parmi le cyclohexane, le méthylcyclohexane, l'heptane, le nonane, le décane, le toluène, le benzène, et l'hexane.

6. Procédé de préparation d'un élastomère à base d'oléfine selon la revendication 1, dans lequel la fourniture d'un flux de recyclage, d'un flux de solvant, et d'un flux de comonomère est mise en œuvre par le mélange du flux de recyclage et du flux de solvant et la fourniture du mélange à la colonne de distillation, et la fourniture directe du flux de comonomère à la colonne de distillation.

7. Procédé de préparation d'un élastomère à base d'oléfine selon la revendication 6, dans lequel le mélange du flux de recyclage et du flux de solvant et la fourniture du mélange à la colonne de distillation fournissent un flux mélangé du flux de recyclage et du flux de solvant à la colonne de distillation via un échangeur de chaleur.

8. Procédé de préparation d'un élastomère à base d'oléfine selon la revendication 1, dans lequel un rapport pondéral du solvant au comonomère oléfinique inclus dans le flux de sortie latéral provenant de la colonne de distillation est de 1,28 à 1,58.

9. Procédé de préparation d'un élastomère à base d'oléfine selon la revendication 1, dans lequel un flux de sortie latéral provenant de la colonne de distillation est produit en sortie depuis une partie latérale à une hauteur de 60 % à 77 % du haut vers le bas de la colonne de distillation.

10. Procédé de préparation d'un élastomère à base d'oléfine selon la revendication 1, dans lequel un rapport de débit massique du comonomère oléfinique au monomère oléfinique fourni au réacteur de polymérisation est de 0,8 à 1,5.

11. Procédé de préparation d'un élastomère à base d'oléfine selon la revendication 1, dans lequel une température dans une partie inférieure de la colonne de distillation est de 120 à 180 °C.

12. Procédé de préparation d'un élastomère à base d'oléfine selon la revendication 1, dans lequel une pression dans une partie supérieure de la colonne de distillation est de 0,7 à 1,0 kg/cm2·g.

13. Procédé de préparation d'un élastomère à base d'oléfine selon la revendication 1, dans lequel un rapport de reflux de tête d'un débit d'un flux de reflux de tête provenant de la colonne de distillation à un débit d'un flux de sortie de tête provenant de la colonne de distillation est de 60 à 75.

14. Procédé de préparation d'un élastomère à base d'oléfine selon la revendication 1, dans lequel un rapport de reflux de fond d'un débit d'un flux de reflux de fond provenant de la colonne de distillation à un débit d'un flux de sortie de fond provenant de la colonne de distillation est de 20 à 220.

15. Procédé de préparation d'un élastomère à base d'oléfine selon la revendication 1, dans lequel la fourniture du flux de sortie latéral provenant de la colonne de distillation et du flux de monomère est mise en œuvre par le mélange du flux de sortie latéral provenant de la colonne de distillation et du flux de monomère et la fourniture d'un mélange du flux de sortie latéral provenant de la colonne de distillation et du flux de monomère au réacteur de polymérisation.
